Europäisches Patentamt

(19) European Patent Office

Office européen des brevets

(11) EP 0 622 071 B1

## (12) EUROPEAN PATENT SPECIFICATION

(45) Date of publication and mention
of the grant of the patent:
**17.06.1998 Bulletin 1998/25**

(51) Int. Cl.⁶: **A61K 7/48**

(21) Application number: **94105941.2**

(22) Date of filing: **16.04.1994**

(54) **Whitening medical preparation**

Medizinisches Bleichmittel

Préparation medicale de blanchissement

(84) Designated Contracting States:
**DE FR GB**

(30) Priority: **28.04.1993 JP 124998/93**

(43) Date of publication of application:
**02.11.1994 Bulletin 1994/44**

(73) Proprietor:
**SNOW BRAND MILK PRODUCTS CO., LTD.**
**Sapporo-shi, Hokkaido 065 (JP)**

(72) Inventors:
• **Itagaki, Yasuharu**
**Sapporo (JP)**
• **Ishikawa, Hidetoshi**
**Nishi-ku, Sapporo (JP)**

• **Kurosawa, Seiji**
**Nishi-ki, Sapporo (JP)**

(74) Representative:
**Patentanwälte**
**Dipl.-Ing. R. Splanemann**
**Dr. B. Reitzner**
**Dipl.-Ing. K. Baronetzky**
**Tal 13**
**80331 München (DE)**

(56) References cited:
**EP-A- 0 334 776**

• **PATENT ABSTRACTS OF JAPAN vol. 16, no. 552
(C-1006) & JP-A-04 210 618 (MORINAGA MILK
IND CO LTD)**

**Description**

Field of the Invention

The present invention relates to the use of angiogenin for the manufacture of a whitening medical preparation. Angiogenin is known to be an angiogenesis factor.

Related Background Art

Angiogenin is known as a material involved in the proliferation of human tumor and the like. The material is one of angiogenesis factors. Human angiogenin was isolated from human tumor cells by Fett et al. (J.W.Fett et al., Biochem., vol.24, 5480-5486,1985). Human angiogenin is known to be a protein of a molecular weight of 14,400. Subsequently the complete amino acid sequence of angiogenin has been determined by Strydom et al. (D.J.Strydom et al., Biochem., vol.24, 5486-5494,1985).

Angiogenin exists in blood and even in milk. Bond et al. have isolated bovine angiogenin from bovine blood (M.D.Bond et al., Biochem. vol. 27, 6282-6287,1988). Maes et al. have isolated angiogenin from bovine milk (P.Maes et al., FEBS LETTERS vol.241, 41-45,1988) and have determined the amino acid sequence of the angiogenin.

JP-A-2-296000 describes a method for recovering angiogenin by adsorbing angiogenin from milk by cation exchange chromatography, and eluating it with an aqueous solution of an alkaline metal salt of a weak organic acid and recovering the eluate by cation exchange chromatography again and gel permeation chromatography. Angiogenin thus obtained has been researched for its physiological activities.

The most important physiological activity of angiogenin is angiogenic activity as described above. Owing to this function, usages of angiogenin for the treatment of injuries, ulcers, organ implantation, circulatory hypofunction have been suggested. However, another functions other than such function have not been known. JP-A-4-210618 describes an example of the use of angiogenin as a hair growth agent.

EP-A-0 334 776 discloses a cosmetic or pharmaceutical composition, to be used on the skin, which composition contains the serum of colostrum which contains angiogenin as the active principle.

On the other hand, blotches and freckles and the like result from melanin production in tissues due to suntan and the like. It is known that melanin may be formed by oxidizing tyrosine catalytically by tyrosinase first and then carrying out several stages of reactions. It has been proved that the development of blotches and freckles may be controlled by inhibiting tyrosinase. Until now the existence of such inhibitory effects in albutin, kojic acid and hydrogenated casein have been confirmed and thus they have been used as skin whitening agents. A variety of materials which may be used as such skin whitening agents are still under investigation.

Disclosure of the Invention

The inventors have investigated the physiological activities of angiogenin, and have first found that angiogenin may control specifically melanin production of B-16 melanoma cells.

Thus the present invention relates to the use of angiogenin as an effective component for the manufacture of whitening medical preparation, Preferred embodiments of the present invention are recited in the dependent claims

The present invention will be described in detail hereinafter.

Angiogenin used as a raw material in the present invention may be derived from any origin or from any species. For example, gene sequences of angiogenin derived from human and bovine sources have been shown and thus angiogenin may be made by a method using genetic recombination. Accordingly, angiogenin produced by the genetic recombination may be used in the present invention. Further, angiogenin is contained in bovine colostrum in a relatively high amount as described above so that angiogenin recovered from milk may be used in the present invention. In addition, angiogenin may be recovered from a culture liquid of a cell culture. JP-A-62-502544 describes an example of angiogenin recovered from a cell culture liquid. In the present invention such kinds of angiogenin may be used.

A method for preparing a skin whitening medical preparation using angiogenin extracted from mammals milk and whitening medical preparations prepared by the method will be described. However, angiogenin used in the present invention should not be limited by angiogenin derived from milk.

An example of a method for obtaining angiogenin from milk will be described hereinafter.

As milk containing angiogenin, colostrum collected from human, bovine, sheep, goat and the like within one to seven days after delivery, especially one to five days after delivery are appropriate since the content of angiogenin fraction is high. However, milk collected from another mammals or normal lactation milk may be used as a raw material in the present invention. In the present invention, these milks may be degreased first, then the milk may be contacted with sulfated polysaccharide having a function of adsorbing lactoferrin and lactoperoxidase(LPO) selectively so that lactoferrin and LPO may be adsorbed to the sulfated polysaccharide. At this point, angiogenin is adsorbed simultaneously.

The sulfated polysaccharide may include sulfonated arginate, sulfonated cellulose, sulfonated dextran, sulfonated chitosan as preferred examples. Sulfonated chitosan is most preferable in the present invention. The sulfonated chitosan is on the market under the name of sulfonated Chitopearl® by Fuji Boseki Co.. Ltd. and thus is easily available. The amount of sulfo groups introduced into the sulfonated Chitopearl is preferably controlled to 10 to 50 µeq/1ml carrier, more preferably to 27 to 40 µeq/1ml carrier.

The adsorption procedures may be conducted using a carrier gel of about 0.5 to 5% by volume, based on the degreased milk volume. The adsorption procedures may be performed by adopting various methods such as batch processes and a method in which degreased colostrum is run through a column which has been filled with a carrier. However, the circulating method using the rotary type column described in Japanese Patent Application(OPI) No.Hei 3-109400 has high adsorption rates and thus is preferable.

The carrier may be then washed with an aqueous solution or buffer solution having an ionic strength of equal or less than 0.2 and a pH of equal or less than 5 to wash out impurities held within the carrier. After washing, the carrier was eluted with an aqueous solution or a buffer solution having an ionic strength of equal or more than 0.5 and a pH of equal or more than 5, preferably having an ionic strength of 0.7 to 2.0 and a pH of 6 to 8 in order to eluate the adsorbed fraction.

The fraction may be desalted since the fraction contains the salts used in the eluation procedures. The desalting procedures may be conducted by dialysis, ion exchange, molecular sieves and the like. Desalting, concentration and purification of the angiogenin-containing fraction may be carried out at the same time by removing a fraction having a molecular weight of equal or less than 50kD by an ultrafiltration membrane. The desalting effects of the ultrafiltration may be ascertained by determining the electrical conductivity and the sulfate ion concentration to serve as indicators. When the electrical conductivity is 400 µs/cm and the sulfate ion concentration is equal or less than 0.29% (based on total solids content), the ultrafiltration may be terminated.

By the above procedures, the angiogenin-containing fraction may be recovered. The fraction may be powdered by drying. However, it is preferable to carry out drying at a temperature as low as possible, for example freeze-drying, in order to prevent deactivation.

The fraction thus obtained may be dissolved in a small amount of Tris-HCl buffer solution (pH 7.0) containing 1 to 5M, preferably 2 to 3M sodium thiocyanate. Sodium thiocyanate is essential to dissolve angiogenin being an effective component of the present invention. The dissolved samples may be subjected to a centrifugation or filtration procedure to remove the impurities, and then cooled ethanol may be added so that the final concentration is reduced to 50 to 80%. After leaving the solution to stand for one hour at -40° C, or for one day and night at equal or less than 5° C, the formed precipitates may be removed. Lactoferrin and LPO which behave similar as angiogenin are transferred to the ethanol precipitation phase, and angiogenin remains in the ethanol phase. After concentration, the angiogenin-containing supernatant may be desalted, such as by dialysis, to recover a fraction containing a high amount of angiogenin. The fraction may be freeze-dried, if necessary. The confirmation that the recovered fraction is angiogenin may be made by determining the inhibitory activity against ribonuclease inhibitor using the method described by Bond et al. (M.D.Bond et al., Analytical Biochemistry, vol.173, 166-173,1988).

Further, the N-terminal amino acid sequence of the fraction obtained may be analyzed using an amino acid sequencer so as to confirm the fraction by the amino acid sequence, if necessary.

The whitening medical preparation used in the present invention contains angiogenin having a concentration of at least 0.001% by weight, preferably 0.01 to 20% by weight. The skin whitening medical preparation may be produced in an appropriate form, such as cream, emulsion, cosmetic lotion and the like by a conventional method using conventional base materials. The whitening medical preparations may contain known optional additives, such as UV absorbing agents, UV scattering agents, melanin synthesis inhibitors, such as albutin, and another pharmaceutically effective components, thickening agents, plasticizers, colorants, flavors and the like. Further, angiogenin has been proved to be neither toxic nor irritating to the skin and to have no side effects.

Effects of the Invention

As described above, the use of angiogenin for the manufacture of skin whitening medical preparation is provided by the present invention. The whitening medical preparation has proved to be safe since it does not have an influence upon the cell growth even with a test using cells, and to have a high whitening effect.

Description of Embodiments

The present invention will be described in detail by reference to the following non-limiting examples.

EXAMPLE 1

Recovery of Angiogenin from Bovine Degreased Milk

(1) Raw bovine milk extracted and collected from normal heifers was subjected to centrifugation to obtain degreased milk, i.e. skim milk. 200L of the degreased milk (cooled to 14° C) was run through a rotary type of absorption device (manufactured by Tokyo Rika Co.. Ltd.) having an internal volume of 1474ml filled with 882ml sulfonated Chitopearl® SU-3 (manufactured by Fuji Boseki Co., Ltd.) in which the amount of sulfonic groups introduced was 38.9 $\mu$eq/ml gel. The running rate was 25kg/hour, and the running was repeated twice. The revolution of the rotary type of reactor was controlled to 30 rpm.

(2) Then 40L warm water at 30 to 35° C was passed through as described in the above step(1) to perform washing. The washing was followed by measuring the absorbance at 280nm using a cell having a light path length of 1cm. When the absorbance was equal or less than 0.01, washing was completed.

(3) After washing, eluation was performed by running through about 67.5L of 0.7M salt solution (prepared by dissolving 0.5mM of sodium bicarbonate, pH7.0). The eluation was complete when absorbance at 280nm was measured using a cell having a light path length of 1cm to be in the range from 0.015 to 0.020. 66.5kg eluate was recovered. The electrical conductivity of the recovered solution was 26.5ms/cm.

(4) The solution obtained in the above step(3) was ultrafiltered to obtain a concentrate using a ultrafiltration device with a UF membrane of 50kD. The electrical conductivity and the concentration of sulfate ions were determined. The concentration procedures were completed at the time when the electrical conductivity was 400 $\mu$S/cm and the concentration of sulfate ions was 0.29% (based on total solids content) to recover a fraction containing angiogenin. The fraction was then freeze-dried to obtain 9.11g dried powder.

(5) 720mg of the fraction containing angiogenin obtained in the above step(4) was dissolved in 100ml of 10mM Tris-HCl buffer solution (pH7.0) containing 3M sodium thiocyanate. After dissolving, the solution was centrifuged for 30 minutes at 1,000 r.p.m. so as to remove insoluble materials.

(6) Then cooled ethanol was added to the supernatant so as to have an ethanol concentration of 80%. The mixture was left to stand at about -40° C for one hour and then centrifuged to remove the precipitates.

(7) After removing the precipitates, the supernatant was transferred to an rotary evaporator for concentration until the liquid amount was reduced to 80ml. The concentrated liquid was then transferred to an dialysis tube, and dialysis was performed against distilled water for one night, and freeze-dried. Thus about 9mg angiogenin was recovered.

EXAMPLE 2

Recovery of Angiogenin from Bovine Colostrum

Bovine milk collected from cows within 5 days after delivery was centrifuged to obtain 100L degreased milk. The subsequent procedures were performed as described in Example 1 to obtain 200mg angiogenin.

EXAMPLE 3

Examples of formulations of medical preparations having skin whitening effect will be shown hereinafter.

(1) Lotion (oily type)

| Sorbitol (70%) | 3.0g |
|---|---|
| Glycerin | 5.0g |
| Angiogenin | 0.2g |
| Water | 70.0g |

To the mixed solution containing the above components, a mixed solution containing

| Allantoin | 0.1g |
|---|---|
| Polyoxyethylene hardened castor oil derivative | 0.5g |
| Ethanol | 20.0g and |
| Flavor | proper quantity |

was added with stirring to make a uniform solution to obtain a lotion having skin whitening effect.

(2) Lotion (dry type)

| Ethylene Glycol | 2.5g |
|---|---|
| Propylene Glycol | 5.0g |
| Silicone | 0.0001g |
| Angiogenin | 0.05g |
| Water | 70.0g |

To the mixed solution containing the above components, a mixed solution containing

| Urea | 1.0g |
|---|---|
| Polyoxyethylene sorbitan monolaurate | 1.2g |
| Ethanol | 20.0g |
| $\varepsilon$-amino caproic acid | 0.1g and |
| Flavor | proper quantity |

was added with stirring to make a uniform solution to obtain a lotion having a skin whitening effect.

(3) Face Washing Cream

| Glycerin | 2.0g |
|---|---|
| Stearic acid | 20.0g |
| Myristic acid | 10.0g |
| Lauric acid | 5.0g |
| Polyoxyethylene laurylether | 1.0g |
| Angiogenin | 0.1g |
| Flavor and Preservative | proper quantity |

The above components were mixed and heated at 75° C to dissolve them, and the mixed solution containing

| Potassium hydroxide | 5.5g and |
|---|---|
| Water | 56.0g |

was added with stirring and cooled to obtain a face washing cream having a skin whitening effect.

(4) Oily Cream

| Beeswax | 10.0g |
|---|---|
| Paraffin wax | 6.0g |
| Lanolin | 3.0g |
| Isopropyl myristate | 6.0g |
| Squalane | 8.0g |
| Liquid paraffin | 25.0g |
| Angiogenin | 0.5g |
| Polyoxyethylene sorbitan monostearate | 1.8g |
| Sorbitan monostearate | 4.2g |
| Preservative | proper quantity |

These components were mixed and heated at 75° C to dissolve them, and the mixed solution containing

| Propylene glycol | 2.0g |
|---|---|
| Boron | 0.7g |
| Urea | 5.0g and |
| Water | 28.0g |

was added with stirring, cooled, and added by flavor to obtain an oily cream having a skin whitening effect.

REFERENCE EXAMPLE

In the reference example, the inhibitory effect on melanin production by angiogenin derived from milk obtained in Example 1 will be illustrated

(1)Biological Activity Measurement

Mouse melanoma B16 cells were seeded on a culture plate having 24 wells at a density of $1 \times 10^5$/ml/well, and incubated for 24 hours under a culture condition of 37° C and 5% $CO_2$. Then 1ml of each angiogenin solution so prepared using Dulbecco Modified Eagle Medium(DMEM) containing 10% fetal bovine serum(FCS) as to have a concentration of 5 μg/ml per well and of an angiogenin solution diluted twice by step dilution with DMEM by using the above solution as a stock solution were added to each well. In addition, albutin which was known to inhibit the melanin production of melanoma cells and to have skin whitening effect was prepared with DMEM so as to have a final concentration of 50 μM, and 1ml of the prepared albutin was added in the same manner as a positive control. Then the culture plate was further incubated for six days under a culture condition of 37° C and 5% $CO_2$. After six days, absorbances at 490nm of the well added with various concentrations of angiogenin and of the well added with albutin were measured, with the

6

well added with only DMEM being used as control. The inhibitory activities on melanin productions of each well were calculated by the following equation:

Inhibitory rate(%) ={1-(absorbance of sample/absorbance of control)} x 100

(2)Inhibitory Activity on Melanin Production

The inhibitory activities on melanin productions of melanoma cells in each well containing various concentrations of angiogenin and albutin will be shown in Table 1.

Table 1

| Sample(conc. per well) | Inhibition Rate(%) |
|---|---|
| Angiogenin(5 $\mu$g/ml) | 41.4 |
| (x1/2) | 59.4 |
| (x1/4) | 56.8 |
| (x1/8) | 41.6 |
| (x1/16) | 43.8 |
| (x1/32) | 7.7 |
| Albutin(50 $\mu$M) | 28.2 |

The angiogenin showed a strong inhibitory effect on melanin production even at a concentration of about 0.15$\mu$ g/ml.

## Claims

1. The use of angiogenin for the manufacture of a medical preparation for whitening skin.

2. The use as claimed in claim 1, wherein said angiogenin is derived from milk.

3. The use as claimed in claims 1 or 2, wherein said angiogenin is derived from bovine milk.

4. The use as claimed in claim 1, wherein said angiogenin is derived from colostrum.

5. The use as claimed in claims 1 or 4, wherein said angiogenin is derived from bovine colostrum.

6. The use as claimed in any one of claims 1 to 5, wherein said preparation contains at least 0.001% by weight angiogenin based on the total amount of said preparation.

7. The use as claimed in claim 6, wherein said preparation contains from 0.01 to 20% by weight angiogenin based on the total amount of said preparation.

8. The use as claimed in any one of claims 1 to 7, wherein said preparation contains a medically acceptable additive and/or carrier.

9. The use as claimed in any one of claims 1 to 8, wherein said preparation contains one or more additives selected from the group consisting of UV absorbing agents, UV scattering agents, melanin synthesis inhibitors, thickening agents, plasticizers, colorants and flavors.

10. The use as claimed in any one of claims 1 to 9, wherein said preparation is in the form of cream, emulsion or lotion.

## Patentansprüche

1. Verwendung von Angiogenin zur Herstellung einer medizinischen Zubereitung zum Weißmachen der Haut.

**2.** Verwendung nach Anspruch 1, wobei das Angiogenin aus Milch stammt.

**3.** Verwendung nach Anspruch 1 oder 2, wobei das Angiogenin aus Rindermilch stammt.

**4.** Verwendung nach Anspruch 1, wobei das Angiogenin aus Kolostrum stammt.

**5.** Verwendung nach Anspruch 1 oder 4, wobei das Angiogenin aus Rinder-Kolostrum stammt.

**6.** Verwendung nach einem der Ansprüche 1 bis 5, wobei die Zubereitung mindestens 0.001 Gew.% Angiogenin, bezogen auf die Gesamtmenge der Zubereitung, enthält.

**7.** Verwendung nach Anspruch 6, wobei die Zubereitung 0.01 bis 20 Gew.% Angiogenin, bezogen auf die Gesamtmenge der Zubereitung, enthält.

**8.** Verwendung nach einem der Ansprüche 1 bis 7, wobei die Zubereitung einen medizinisch akzeptablen Zusatz und/oder einen Träger enthält.

**9.** Verwendung nach einem der Ansprüche 1 bis 8, wobei die Zubereitung einen oder mehrere Zusätze, ausgewählt aus der Gruppe, bestehend aus UV-Absorptionsmitteln, UV-Licht streuenden Mitteln, Melanin-Syntheseinhibitoren, Verdikkungsmitteln, Weichmachern, Farbstoffen und Aromastoffen ausgewählt ist.

**10.** Verwendung nach einem der Ansprüche 1 bis 9, wobei die Zubereitung in Form einer Creme, Emulsion oder Lotion vorliegt.

## Revendications

**1.** Utilisation d'angiogénine pour la fabrication d'une préparation à usage médical pour blanchir la peau.

**2.** Utilisation selon la revendication 1, dans laquelle ladite angiogénine dérive du lait.

**3.** Utilisation selon les revendications 1 ou 2, dans laquelle ladite angiogénine dérive du lait de bovin.

**4.** Utilisation selon la revendication 1, dans laquelle ladite angiogénine dérive du colostrum.

**5.** Utilisation selon les revendications 1 ou 4, dans laquelle ladite angiogénine dérive du colostrum de bovin.

**6.** Utilisation selon l'une quelconque des revendications 1 à 5, dans laquelle ladite préparation contient au moins 0,001 % en poids d'angiogénine par rapport à la quantité totale de ladite préparation.

**7.** Utilisation selon la revendications 6, dans laquelle ladite préparation contient de 0,01 à 20 % en poids d'angiogénine par rapport à la quantité totale de ladite préparation.

**8.** Utilisation selon l'une quelconque des revendications 1 à 7, dans laquelle ladite préparation contient un additif et/ou un excipient acceptable d'un point de vue médical.

**9.** Utilisation selon l'une quelconque des revendications 1 à 8, dans laquelle ladite préparation contient un ou plusieurs additifs choisis dans l'ensemble comprenant les agents absorbant les UV, les agents diffusant les UV, les inhibiteurs de la synthèse de la mélanine, les agents épaississants, les plastifiants, les colorants et les aromatisants.

**10.** Utilisation selon l'une quelconque des revendications 1 à 9, dans laquelle ladite préparation se présente sous forme d'une crème, d'une émulsion ou d'une lotion.